# EUROPÄISCHE PATENTSCHRIFT

(11) **EP 2 227 477 B1**
(45) Veröffentlichungstag und Bekanntmachung des Hinweises auf die Patenterteilung: **28.09.2011**
(21) Anmeldenummer: 08854379.8
(22) Anmeldetag: 25.11.2008
(51) Int. Cl.: C07F 7/00, C07F 11/00

(54) **ORTHO-METALLIERTE, CHELATSTABILISIERTE BENZYLAMINE DER SELTENERDMETALLE (SE)**
ORTHO-METALATED, CHELATE-STABILIZED BENZYLAMINES OF THE RARE EARTH METALS (RE)
BENZYLAMINES DES MÉTAUX DE TERRES RARES, STABILISÉES PAR DES CHÉLATES ORTHO-MÉTALLÉES

(30) Priorität: 28.11.2007 DE 102007057586
(43) Veröffentlichungstag der Anmeldung: 15.09.2010
(73) Patentinhaber: Philipps-Universität Marburg, 35037 Marburg (DE)
(72) Erfinder: SUNDERMEYER, Jörg, 35041 Marburg (DE); PETROV, Alexander, 38108 Braunschweig (DE); THOMAS, Oliver, 51069 Köln (DE)
(74) Vertreter: Buchhold, Jürgen
(86) Internationale Anmeldenummer: PCT/DE2008/001941
(87) Internationale Veröffentlichungsnummer: WO 2009/067999

(56) Entgegenhaltungen:
- DENIS GRIBKOV: "Doktorarbeit: Novel Catalysts for Stereoselective Hydroamination of Olefins Based on Rare Earth and Group IV Metals" Juli 2005 (2005-07), UNIVERSITÄT ERLANGEN , ERLANGEN , XP002522405 Gefunden im Internet: URL:http://deposit.ddb.de/cgi-bin/dokserv? idn=976595567&dok_var=d1&dok_ext=pdf&filen ame=976595567.pdf> Seite 72 das ganze Dokument

## Beschreibung

Die vorliegende Erfindung betrifft die Gebiete Seltenerdmetallchemie, Komplexchemie, Metallorganyle und Polymerisationskatalysatoren.

### Stand der Technik

Die Addition von Aminen RR'NH an Olefine (Hydroaminierung) läuft nur über geeignete Katalysatoren ab. Eine der ganz großen Herausforderungen ist die Steigerung der Katalysatoreffizienz insbesondere in der intermolekularen Variante. Ein effizienter und thermisch stabiler Katalysator wäre wirtschaftlich höchst interessant. (Denis Gimbkov : "Doktorarbeit : Novel catalysts for Stereoselective Hydroanimation of Olefins based on Rare Earth and Giroun IV Metals, Juli 2005, Universität Erlangen).

Es besteht ein großes Interesse seitens der Anwender im Labor und in der Chemischen Industrie an in Lösung lagerfähigen und dennoch hochreaktiven SE-Organylen. Dieses Ziel ist bislang nicht erreicht: Während beispielsweise Phenyllithium (PhLi) ein lagerfähiges, kommerziell verfügbares, sehr probates Metallierungsreagenz darstellt, sind beispielsweise Verbindungen [(SE)Ph₃(THF)₃] (SE = Y, La, Ce, Sm) selbst bei tiefen Temperaturen instabil - dabei wären sie recht kostengünstig und hätten als Ummetallierungsreagenzien und Katalysatorvorstufen ein hohes Anwendungspotential.

Offensichtlich sind nur wenige SE-Verbindungen auf Basis des Liganden N,N-Dimethylbenzylamin bekannt. Es sind insbesondere diejenigen SE-Trisaryle, deren Kation einen relativ geringen Ionenradius besitzen. Den geringsten lonenradius besitzen Sc und Lu (Hollemann Wiberg, Lehrbuch der Anorganischen Chemie, 102. Auflage, W. de Gruyter 2007).

Wegen ihrer thermischen Instabilität und Neigung zur Zersetzung bei Raumtemperatur sind bis heute lediglich ortho-lithierte tris-[N,N-Dimethylbenzylamin]-Komplexe von Y, Sc, Er, Yb und Lu bekannt (Organometallics 1985, 4, 1440-1444; Organometallics 1984, 3, 939-941; J. Am. Chem. Soc., 1978, 100, 8068-8073; Inorg. Synth. 1989, 26, 150ff; J. Organomet. Chem. 1989, 364, 79-86).

Der Fachmann kennt außerdem ortho-lithierte Komplexe von einiger weiterer N,N-Dimethylbenzylderivate (Acta Chem. Scand. 1963, 17, 1735-1742; J. Am. Chem. Soc., 1928, 50, 1152; Chem. Ber. 1941,74B, 982-986; Chem. Eur. J. 2005, 11, 253-261; Tetrahedron, 1989, 45, 569-578; Organometallics 2000, 19, 206; Bull. Chem. Soc. Jpn. 1999, 72, 1879). SE-Aryle dieser substituierten Derivate sind gänzlich unbekannt.

Bekanntlich erfolgt die Deprotonierung von racemischen und chiralen [1-(Dimethylamino)ethyl]benzol ausschließlich in ortho-Position des aromatischen Rings. Es wurden insbesondere durch die Arbeitsgruppe *van Koten* zahlreiche organische Reaktionen von [1-(Dimethylamino)ethyl]benzol mit Elektrophilen beschrieben [Referenzen: a) G. van Koten, J. T. B. H. Jastrzebski, J. G. Noltes, W. M. G. F. Pontenagel, J. S. Kroon, A. Spek, J. Am. Chem. Soc. 1978, 100, 5021. b) C. M. P. Kronenburg, E. Rijnberg, J. T. B. H. Jastrzebski, H. Kooijman. A. Spek, G. van Koten, Eur. J. Org. Chem. 2004, 153. c) C. M. P. Kronenburg, E. Rijnberg, J. T. B. H. Jastrzebski, H. Kooijman. M. Lutz, A. Spek, R. Gossage, G. van Koten, Chem. Eur. J. 2005, 11, 253]. Ein anderes Lithiumaryl basierend auf der Vorstufe N,N-Dimethylcumylamin - 2-(*N*,*N*,α,α-Tetramethyl-aminomethyl)-phenyllithium (3-Li) - wurde ein erstes Mal durch Brom-Lithium-Austausch des entsprechenden ortho-Bromaryls mit *n*-BuLi *in situ* erzeugt und in einer Folgereaktion verwendet [Referenzen: a) M. Asakura, M. Oki, S. Toyota, Organometallics 2000, 19, 206. b) S. Toyota, M. Asakura, T. Futawaka, M. Oki, Bull. Chem. Soc. Jpn. 1999, 72, 1879]. Uns gelang die Synthese von N,N-Dimethylcumylamin-Li durch *ortho-*Metallierung mit *t*-BuLi bei RT in Pentan. Das Produkt N,N-Dimethylcumylamin-Li lässt sich bei -30°C durch Kristallisation in 65% Ausbeute isolieren. Einkristalle konnten aus Ether gewonnen werden.

Ortho-metallierte Benzylamine mit R¹ = R² = H wurden bereits vereinzelt als Liganden in der SE-Organometallchemie kleiner Kationen (z.B. Sc, Lu) eingesetzt [L.E. Manzer, J. Am. Chem. Soc. 1978, 100, 8068-8073. b) A.L. Wayda, Organometallics 1984, 3, 939-941. c) A.L. Wayda, Organometallics 1985, 4, 1440-1444]. Die Produkte wurden in nur wenigen Fällen kristallstrukturanalytisch charakterisiert, da Verbindungen mit R¹ = R² = H insbesondere für größere Kationen thermisch instabil sind.

Die bisher bekannten Ligandkomplexe unterliegen dem thermischen Zerfall auf Grund eines H-Shifts:

Thermischer Zerfallsweg am Beispiel der literaturbekannten N,N-Dimethylbenzylamin-Ligandkomplexen Ln(dmba)₃:

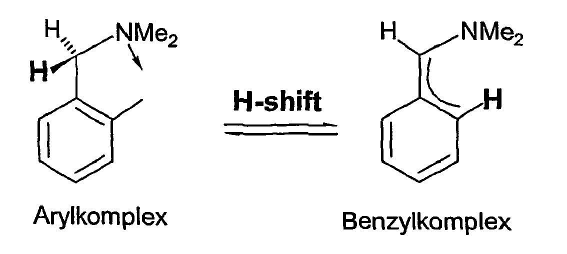

Die kostengünstige direkte Gewinnung C,N-chelatstabilisierter, ortho-metallierter Tris-aryl-SE-Verbindungen wäre jedoch von großem Interesse. Die vorliegende Erfindung überwindet die Nachteile des Standes der Technik, indem sie neue, ortho-metallierte Benzylaminkomplexe der Seltenerdmetalle und Verfahren zu deren Herstellung bereitstellt. Bei den erfindungsgemäßen ortho-metallierte Benzylamine ist der deren Zerfallsweg blockiert, so dass sie über lange Zeit haltbar sind.

### Aufgabe

Aufgabe der Erfindung ist es, neue ortho-metallierte Benzylaminkomplexe der Seltenerdmetalle und Verfahren zu ihrer Herstellung bereitszustellen.

### Lösung der Aufgabe

Die Aufgabe, neue, ortho-metallierte Benzylaminkomplexe der Seltenerdmetalle und Verfahren zu ihrer Herstellung bereitszustellen, wird erfindungsgemäß gelöst durch homoleptische Komplexe gemäß Formel (I): 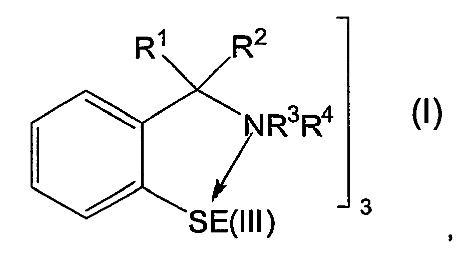 worin
- SE ein Seltenerdmetall darstellt, ausgewählt aus Sc, Y, La, Ce, Pr, Nd, Pm, Sm, Eu, Gd, Tb, Dy, Ho, Er, Tm, Yb, Lu,
- R¹ und R² unabhängig voneinander ausgewählt sind aus Wasserstoff, einer unverzweigten oder verzweigten Alkylgruppe mit 1 bis 10 Kohlenstoffatomen oder einer Arylgruppe
   oder
   R¹ und R² gemeinsam für 1,ω-Alkyldiylgruppe, ausgewählt aus 1,4-Butandiyl und 1,5-Pentandiyl stehen,
- R³ und R⁴ unabhängig voneinander ausgewählt sind aus einer unverzweigten oder verzweigten Alkylgruppe mit 1 bis 10 Kohlenstoffatomen, einer Arylgruppe oder einer Trialkylsilylgruppe -SiR⁵, wobei R⁵ eine unverzweigte oder verzweigte Alkylgruppe mit 1 bis 10 Kohlenstoffatomen darstellt,
   und wobei
   mindestens einer der beiden Reste R¹ und R² eine von Wasserstoff verschiedene Gruppe ist.

Überraschend wurde gefunden, dass die unerwünschte thermische Instabilität der Trisaryl-Verbindungen drastisch abnimmt, wenn mindestens einer der Substituenten R¹ und R² kein Wasserstoffatom darstellt. Bereits durch die Substitution eines benzylischen Protons durch eine Alkyl- oder Arylgruppe lässt sich die thermische Stabilität steigern, noch deutlicher jedoch durch Substitution beider Protonen.

Unter dem Begriff "Seltenerdmetalle" werden in der vorliegenden Erfindung die Metalle Sc, Y und La der III. Gruppe des Periodensystems sowie die Lanthanoide verstanden. Lanthan (La) ist einerseits ein Metall der III. Gruppe. Andererseits ist es aber auch der erste Vertreter der nach ihm benannten Gruppe der 4f-Elemente, nämlich der Lanthanoide. Im Rahmen der vorliegenden Erfindung wird La der III. Gruppe zugerechnet, und unter den "Lanthanoiden", welche die Zentralatome der erfindungsgemäßen Komplexe darstellen, werden die Metalle Ce, Pr, Nd, Pm, Sm, Eu, Gd, Tb, Dy, Ho, Er, Tm, Yb und Lu verstanden.

Falls R¹, R², R³, R⁴ und/oder R⁵ unabhängig voneinander für eine Alkylgruppe stehen, so ist diese bevorzugt ausgewählt aus Methyl, Ethyl, n-Propyl, iso-Propyl, n-Butyl, 2-Butyl, tert.-Butyl.

Falls R¹, R², R³ und R⁴ unabhängig voneinander für eine Arylgruppe stehen, so handelt es sich bevorzugt um eine Phenylgruppe.

In einer bevorzugten Ausführungsform sind R¹ und R² unabhängig voneinander ausgewählt aus aus einer unverzweigten oder verzweigten Alkylgruppe mit 1 bis 10 Kohlenstoffatomen und einer Arylgruppe.

In einer weiteren bevorzugten Ausführungsform stehen R¹ und R² gemeinsam für 1,ω-Alkyldiylgruppe, ausgewählt aus 1,4-Butandiyl und 1,5-Pentandiyl.

Im Rahmen der vorliegenden Erfindung wurde festgestellt, dass die thermische Instabilität der bisher bekannten Benzylamine der Seltenerdmetalle darauf zurückzuführen ist, dass gerade für die SE-Metalle mit größerem Ionenradius ein SE-Benzylanion etwas stabiler ist als ein SE-Aryl-Anion. Es sollte also zu einer prototropen Verschiebung des benzylischen Protons auf das Arylanion kommen, insbesondere wenn ein besonders acides sekundäres benzylisches Proton wie in Liganden von Typ des [1-(Dimethylamino)ethyl]benzols vorhanden ist.

In der vorliegenden Erfindung werden daher erstmalig SE-Verbindungen beschrieben, bei denen entweder R¹= H und R² ≠ H ist oder sowohl R¹ und R² ≠ H sind.

Es zeigte sich, dass erstere eine deutlich erhöhte thermische Stabilität im Vergleich zu instabilen, daher bislang unbekannten Derivaten mit R¹ = R² = H besitzen, und dass insbesondere letztere mit R¹ und R² = ≠ H kristalline, bei Raumtemperatur stabile Trisaryle gerade auch für große Kationen SE = Nd, Sm liefern, die sich sonst dieser Synthesestrategie entzogen:

Durch Substitution mindestens eines der beiden benzylischen Protonen, besser beider benzylischer Protonen R¹ bzw. R² durch Alkylgruppen bzw. Arylgruppen wird einer der möglichen Zerfallswege, die prototrope Umlagerung (H-shift) eines Arylkomplexes in einen Benzylkomplex, erschwert bzw. gänzlich blockiert:

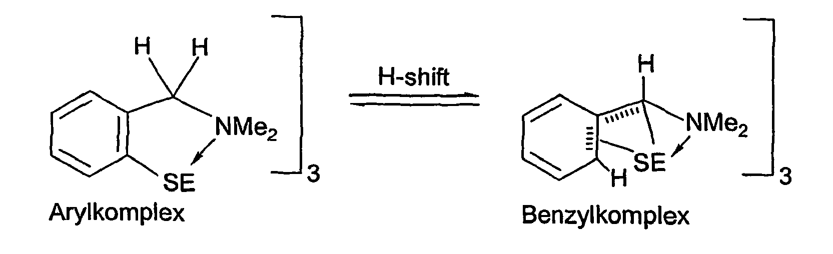

Dabei steht ME für eine Methylgruppe.

Im Einklang mit dieser Annahme ist auch, dass einige der bisher bekannten Arylkomplexe mit R¹ = R² = H sich in Toluol (als benzylisch CH-acides Lösungsmittel und Substrat) zersetzen.

Die Aufgabe, ein Verfahren zur Herstellung der erfindungsgemäßen Komplexe bereitzustellen, wird erfindungsgemäß gelöst durch ein Verfahren umfassend die Schritte:
- Zugabe von 3 Äquivalenten des ortholithiierten Arylliganden zu einer Suspension von 1 Äquivalent des wasserfreien SE-Halogenids in einem absoluten Ether bei Raumtemperatur und unter Schutzgasatmosphäre,
- Rühren für 1 Stunde,
- Isolation und Reinigung des erfindungsgemäßen homoleptischen Komplexes.

Das erfindungsgemäße Verfahren wird bei Raumtemperatur unter Schutzgasatmosphäre und ausgeheizten Glasgeräten durchgeführt.

Bei dem Seltenerdmetall-Halogenid (SE-Halogenid) handelt es sich um ein Fluorid, Chlorid, Bromid oder Iodid, wobei Chloride bevorzugt sind.

Als Schutzgas eignen sich beispielsweise Argon, Helium, Stickstoff und Gemische dieser Gase. Bevorzugt ist Argon.

Der Ether wird ausgewählt aus Diethylether, THF, Dimethylether und Dimethoxyethan (DME).

Isolation und Reinigung der erfindungsgemäßen Komplexe können beispielsweise erfolgen, indem nach beendetem Rühren bis zur Trockne eingeengt, der Rückstand in Toluol (abs.) aufgenommen und über Celite filtriert wird. Das Lösungsmittel wird entfernt und die zurückgebliebene Substanz in Hexan (abs.) unter leichtem Erwärmen aufgelöst. Nach kurzem Abkühlen tritt sofortige Kristallisation ein.

Diese erfindungsgemäßen Verbindungen können als Katalysatoren für die Hydroaminierung von Olefinen verwendet werden. Der besondere Vorteil thermisch stabiler Katalysatoren ist ihre Langlebigkeit (hohe Turn Over Number) und die Möglichkeit, durch Temperaturerhöhung die Reaktionsgeschwindigkeit (Turn Over Frequency) zu erhöhen. Weiterhin können erstmalig auch Komplexe von SE-Metallen wie etwa Nd, Sm und Gd, für die aufgrund ihrer thermischen Empfindlichkeit bislang keine Trisaryle beschrieben waren, als effiziente Hydroaminierungskatalysatoren eingesetzt werden.

### Ausführungsbeispiele

### Ausführungsbeispiel 1:

### Synthese des N,N-Dimethylcumylamin 3

Die Synthese des Cumylamins wurde nach Literaturvorschrift durchgeführt. (J. of org. chem. 2007, 72, 9, 3193 - 3206, Supporting Informations). Die Methylierung erfolgte nach der Methode von ESCHWEILER-CLARKE.
Zu 29.6 mL konzentrierter Ameisensäure (0.784 mol, 8 eq) wurden 16 g des Cumylamins (0.098 mol, 1 eq) bei 0 °C langsam zugetroft. Das Reaktionsgemisch wurde mit 34.2 mL 37%iger wässeriger Formaldehyd (0.323 mol, 3.3 eq) versetzt und auf 70 °C erhitzt. Die auftretende Gasentwicklung wurde mittels Blasenzähler verfolgt. Nach Reaktionsende wurde das Gemisch abgekühlt und mit Natronlauge alkalisch gemacht. Die wässerige Lösung wurde mit 3 mal 80 mL Et₂O extrahiert, die organische Phase mit MgSO₄ getrocknet und das Lösungsmittel entfernt. Das erhaltene Rohprodukt wurde durch Destillation aufgereinigt. (81 - 83 °C, 7 mm Hg).
Ausbeute: 12.2 g / 76 %

### Ausführungsbeispiel 2:

### Lithiierung des N,N-Dimethylcumylamins 3

Zu einer Lösung aus 12.2 g N,N-Dimethylcumylamin (0.075 mol, 1 eq) und 250 mL Hexean (abs.) wurden langsam 60 mL einer 1.5 molaren tBuLi Lösung in Pentan (0.090 mol, 1.2 eq) zugegeben. Das Reaktionsgemisch wurde 48h bei Raumtemperatur gerührt. Nach Filtration und waschen mit 30 mL Hexan wurde ein leicht gelblicher Feststoff erhalten.
Ausbeute: 7.5 g / 60 %

### Ausführungsbeispiel 3:

### Herstellung [Y(C₆H₄CH₂N(CH₃)₂)₃]

Vergleichsprobe, bekannte Verbindung, siehe Organometallics 2004, 23, 2601-2612.

### NMR

¹H-NMR (300 MHz, C₆D₆): δ = 2.15 (s, 6H), 3.44 (s, 2H), 6.96 (d, 1H), 7.25 (dt, 1H), 7.34 (t, 1H), 8.19 (d, 1H) ppm.

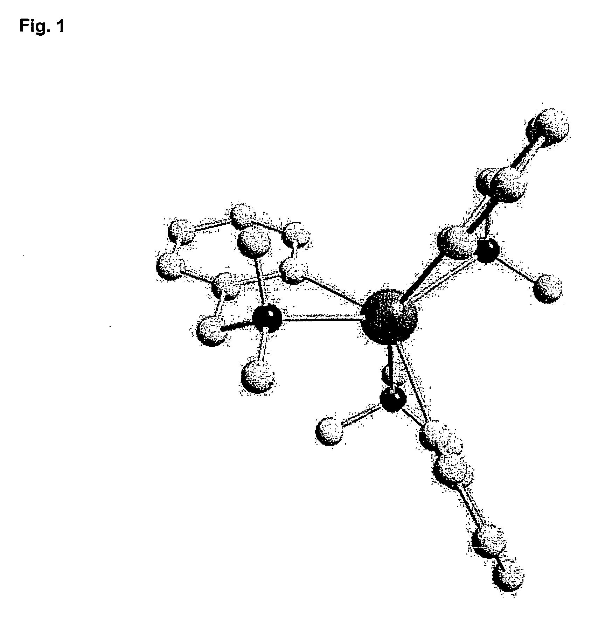

Die Kristallstruktur ist in Fig. 1 gezeigt.

### Ausführungsbeispiel 4:

### Herstellung von [Y(C₆H₄CH(CH₃)N(CH₃)₂)₃]

Die Herstellung erfolgte nach der erfindungsgemäßen allgemeinen Vorschrift.

### NMR

¹H-NMR (300 MHz, C₆D₆): δ = 1.20 (d, 3H), 2.5 (sb, 6H), 3.19 (sb, 1H), 7.00 (t, 1H), 7.25 (dt, 1H), 7.34 (t, 1H), 8.32 (sb, 1H) ppm.

### CHN-Analyse

| | **Berechnet** | **Gefunden** |
|---|---|---|
| **N** | 7.88 | 7.85 |
| **C** | 67.53 | 66.98 |
| **H** | 7.93 | 7.84 |

### Ausführungsbeispiel 5:

### Herstellung von [Dy(C₆H₄CH(CH₃)N(CH₃)₂)₃]

Die Herstellung erfolgte nach der erfindungsgemäßen allgemeinen Vorschrift.

### CHN-Analyse

| | **Berechnet** | **Gefunden** |
|---|---|---|
| **N** | 6.92 | 6.81 |
| **C** | 59.34 | 58,69 |
| **H** | 6.97 | 6.89 |

### Ausführungsbeispiel 6:

### Herstellung von [Nd(C₆H₄CH(CH₃)N(CH₃)₂)₃]

Die Herstellung erfolgte nach der erfindungsgemäßen allgemeinen Vorschrift.

### CHN-Analyse

| | **Berechnet** | **Gefunden** |
|---|---|---|
| **N** | 7.13 | 6.84 |
| **C** | 61.18 | 58.91 |
| **H** | 7.19 | 7.00 |

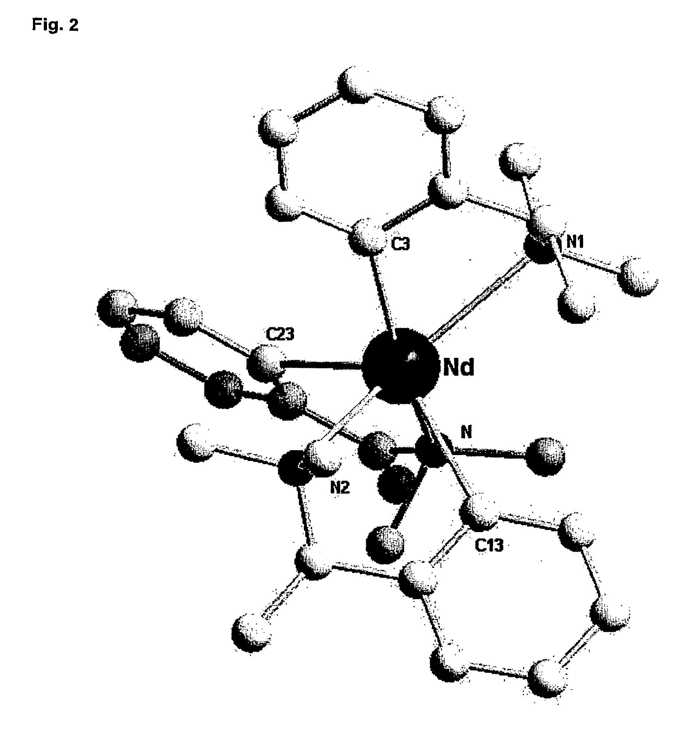

Die Kristallstruktur ist in Fig. 2 gezeigt.

### Ausführungsbeispiel 7:

### Herstellung von [Sm(C₆H₄C(CH₃)₂N(CH₃)₂)₃]

Die Herstellung erfolgte nach der erfindungsgemäßen allgemeinen Vorschrift.

### CHN-Analyse

| | **Berechnet** | **Gefunden** |
|---|---|---|
| **N** | 6.60 | 6.49 |
| **C** | 62.21 | 61.18 |
| **H** | 7.59 | 7.92 |

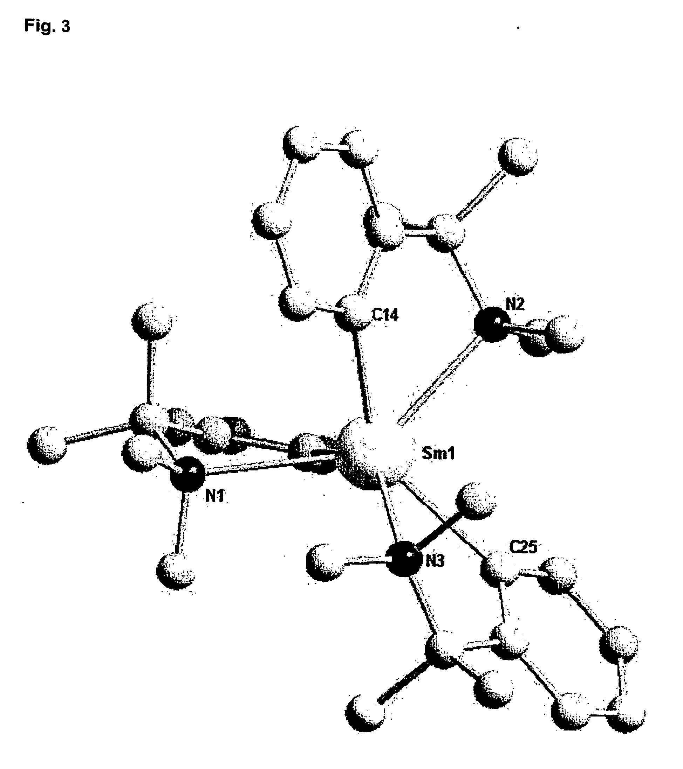

Die Kristallstruktur ist in Fig. 3 gezeigt.

### Ausführungsbeispiel 8:

### Herstellung von [Gd(C₆H₄C(CH₃)₂N(CH₃)₂)₃]

Die Herstellung erfolgte nach der erfindungsgemäßen allgemeinen Vorschrift.

### CHN-Analyse

| | **Berechnet** | **Gefunden** |
|---|---|---|
| **N** | 6.52 | 6.45 |
| **C** | 61.55 | 61.05 |
| **H** | 7.51 | 7.40 |

### Ausführungsbeispiel 9:

### Katalyseexperimente zur Hydroaminierung

Die Experimente wurden im NMR-Maßstab durchgeführt. Auf katalytische Aktivität wurden beispielhaft die Verbindungen [Y(C₆H₄CH₂N(CH₃)₂)₃] (bekannte Vergleichsprobe), [Y(C₆H₄CH(CH₃)N(CH₃)₂)₃] (neu) und [Gd(C₆H₄C(CH₃)₂N(CH₃)₂)₃] (neu) geprüft.

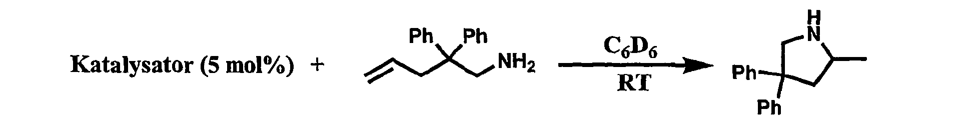

In einer Glovebox wurden 5 mol% des Katalysators mit einer Lösung des Substates in D₆-Benzol versetzt. Die Zeitmessung startete bei Zugabe des Substrates. Verfolgt wurde der Reaktionsfortschritt bei 25°C.

### Ergebnisse:

| | ***Quantitativer Umsatz nach*** | ***TOF*** |
|---|---|---|
| ***[Y(C₆H₄CH₂N(CH₃)₂)₃]*** | 46 min | 26.6 |
| ***[Y(C₆H₄CH(CH₃)N(CH₃)₂)₃]*** | 21 min | 57.1 |
| ***[Gd(C₆H₄C(CH₃)₂N(CH₃)₂)₃]*** | 15 min | 80.0 |

Diese Experimente können als Proof of Concept angesehen werden, dass die neuen beanspruchten Verbindungen mit Substituenten R1 und R2 ungleich H
1) zu Katalysatoren mit deutlich höherer Aktivität führen (Eintrag 2, Y-Komplex).
2) thermisch stabiler sind, so dass auch katalytisch wirksame Seltenerdmetallkomplexe existent sind (z.B. Gd), für die es bislang kein Pendant mit R¹ = R² = H gibt (Eintrag 3).

### Abbildungslegenden

**Fig. 1**
   Struktur von [Y(C₆H₄CH₂N(CH₃)₂)₃],
   monoklin, C 1 2/c 1, wR2 = 0.1048, R1 = 0.0415
**Fig. 2**
   Struktur von [Nd(C₆H₄CH(CH₃)N(CH₃)₂)₃]
   Orthorhomisch, P 2₁ 2₁ 2₁, Z = 4, wR2 = 0.0699, R1 = 0.0291
**Fig. 3**
   Struktur von Sm(C₆H₄C(CH₃)₂N(CH₃)₂)₃]
   Monoklin, P 2₁/c, Z = 4, wR2 = 0.0522, R1 = 0.0332

## Patentansprüche

1. Homoleptische Komplexe gemäß Formel (I): 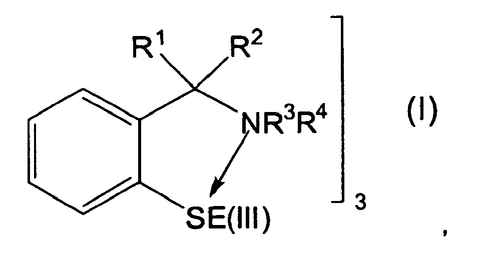 worin
- SE ein Seltenerdmetall darstellt, ausgewählt aus Sc, Y, La, Ce, Pr, Nd, Pm, Sm, Eu, Gd, Tb, Dy, Ho, Er, Tm, Yb, Lu,
- R¹ und R² unabhängig voneinander ausgewählt sind aus Wasserstoff, einer unverzweigten oder verzweigten Alkylgruppe mit 1 bis 10 Kohlenstoffatomen oder einer Arylgruppe, wobei mindestens einer der Substituenten R¹ und R² kein Wasserstoffatom darstellt,
- oder
R¹ und R² gemeinsam für 1,ω-Alkyldiylgruppe, ausgewählt aus 1,4-Butandiyl und 1,5-Pentandiyl stehen,
- R³ und R⁴ unabhängig voneinander ausgewählt sind aus einer unverzweigten oder verzweigten Alkylgruppe mit 1 bis 10 Kohlenstoffatomen, einer Arylgruppe oder einer Trialkylsilylgruppe -SiR⁵, wobei R⁵ eine unverzweigte oder verzweigte Alkylgruppe mit 1 bis 10 Kohlenstoffatomen darstellt,
und wobei
mindestens einer der beiden Reste R¹ und R² eine von Wasserstoff verschiedene Gruppe ist.

2. Homoleptische Komplexe gemäß Anspruch 1, **dadurch gekennzeichnet, dass** R¹ und R² unabhängig voneinander ausgewählt sind aus einer unverzweigten oder verzweigten Alkylgruppe mit 1 bis 10 Kohlenstoffatomen oder einer Arylgruppe.

3. Homoleptische Komplexe gemäß Anspruch 1, **dadurch gekennzeichnet, dass** R¹ und R² gemeinsam für 1,ω-Alkyldiylgruppe, ausgewählt aus 1,4-Butandiyl und 1,5-Pentandiyl stehen.

4. Verfahren zur Herstellung der erfindungsgemäßen Komplexe, umfassend die Schritte:
- Zugabe von 3 Äquivalenten des ortholithiierten Arylliganden zu einer Suspension von 1 Äquivalent des wasserfreien SE-Halogenids in einem absoluten Ether bei Raumtemperatur und unter Schutzgasatmosphäre,
- Rühren für 1 Stunde,
- Isolation und Reinigung des erfindungsgemäßen homoleptischen Komplexes.

5. Verwendung von Komplexen gemäß einem der Ansprüche 1 bis 3 als Katalysatoren für die Hydroaminierung von Olefinen.

## Claims

1. Homoleptic complexes according to formula (I): 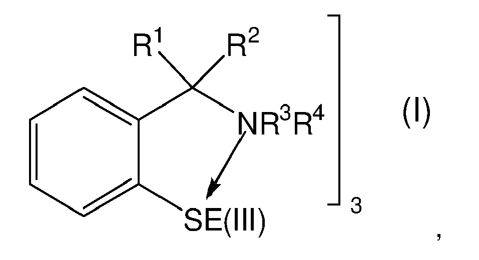 wherein
- SE represents a rare-earth metal selected from Sc, Y, La, Ce, Pr, Nd, Pm, Sm, Eu, Gd, Tb, Dy, Ho, Er, Tm, Yb, Lu,
- R¹ und R² are selected, independently of one another, from hydrogen, a linear or branched alkyl group with 1 to 10 carbon atoms or an aryl group, wherein at least one of both substituents R¹ and R² does not represent a hydrogen atom,
- or
R¹ and R² together stand for 1,ω-alkyldiyl group, selected from 1,4-butandiyl and 1,5-pentandiyl,
- R³ and R⁴ are selected, independently of one another, from a linear or branched alkyl group with 1 to 10 carbon atoms, an aryl group or a trialkylsilyl group -SiR⁵, wherein R⁵ represents a linear or branched alkyl group with 1 to 10 carbon atoms,
and wherein
at least one of both residues R¹ and R² is a group different from hydrogen.

2. Homoleptic complexes according to claim 1, wherein R¹ and R² are selected, independently of one another, from a linear or branched alkyl group with 1 to 10 carbon atoms or an aryl group.

3. Homoleptic complexes according to claim 1, wherein R¹ and R² together stand for 1,ω-alkyldiyl group, selected from 1,4-butandiyl and 1,5-pentandiyl.

4. Method for the production of the complexes according to the present invention, comprising the steps:
- addition of 3 equivalents of the ortho-lithiated aryl ligand to a suspension of 1 equivalent of the anhydrous SE halide in an absolute ether at room temperature and under inert gas atmosphere,
- stirring for 1 hour,
- isolation and purification of the homoleptic complexe according to the present invention.

5. Use of complexes according to one of claims 1 to 3 as catalysts for the hydroamination of olefins.

## Revendications

1. Complexes homoléptiques selon la formule (I): 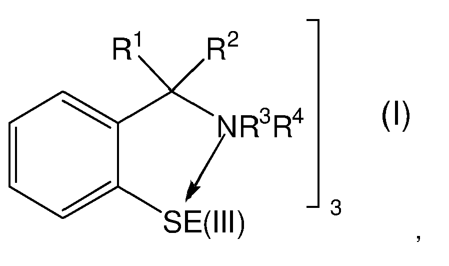 où
- SE représente un métal des terres rares, sélectionné de Sc, Y, La, Ce, Pr, Nd, Pm, Sm, Eu, Gd, Tb, Dy, Ho, Er, Tm, Yb, Lu,
- R¹ et R² sont sélectionnés indépendamment l'un de l'autre d'hydrogène, d'un groupe alkyle linéaire ou ramifié avec 1 à 10 atomes de carbone ou un groupe aryle, où au moins l'un des deux substituants R¹ et R² ne représente pas un atome d'hydrogène,
- ou
R¹ et R² représentent ensemble un 1,ω-groupe alkyldiyle, sélectionnés de 1,4-butanediyle et 1,5-pentanediyle,
- R³ et R⁴ sont sélectionnés indépendamment l'un de l'autre d'un groupe alkyle linéaire ou ramifié avec 1 à 10 atomes de carbone, d'un groupe aryle ou d'un groupe trialkylsilyle-SiR⁵, où R⁵ représente un groupe alkyle linéaire ou ramifié avec 1 à 10 atomes de carbone,
et où au moins l'un des deux restes R¹ et R² est un groupe différent de l'hydrogène.

2. Complexes homoléptiques selon la revendication 1, **caractérisés en ce que** R¹ et R² sont sélectionnés indépendamment l'un de l'autre d'un groupe alkyle linéaire ou ramifié avec 1 à 10 atomes de carbone ou d'un groupe aryle.

3. Complexes homoléptiques selon la revendication 1, **caractérisés en ce que** R¹ et R² représentent ensemble un 1,ω-groupe alkyldiyle, sélectionnés de 1,4-butanediyle et 1,5-pentanediyle.

4. Procédé pour la production du complexe selon l'invention comprenant les étapes :
- Addition de 3 équivalents du ligand d'aryle ortho-lithié à une suspension d'1 équivalent du SE-halogénure anhydre dans un éther absolu à température ambiante et sous atmosphère de gaz protecteur,
- agitation pendant 1 heure,
- isolation et nettoyage du complexe homoléptique selon l'invention.

5. Utilisation de complexes selon l'une des revendications 1 à 3 en tant que catalyseurs pour l'hydroamination d'oléfines.
